Europäisches Patentamt

(19) European Patent Office

Office européen des brevets

(11) Publication number: **0 351 686**
**A2**

## (12) EUROPEAN PATENT APPLICATION

(21) Application number: **89112592.4**

(22) Date of filing: **10.07.89**

(51) Int. Cl.⁴: **A61K 9/00 , A61K 37/18**

(30) Priority: **22.07.88 IT 2145988**

(43) Date of publication of application:
**24.01.90 Bulletin 90/04**

(84) Designated Contracting States:
**AT BE CH DE ES FR GB GR IT LI LU NL SE**

(71) Applicant: **Torre, Alberto**
**Viale E. Forlanini, 15**
**I-20134 Milano(IT)**

(72) Inventor: **Torre, Alberto**
**Viale E. Forlanini, 15**
**I-20134 Milano(IT)**

(74) Representative: **Bianchetti, Giuseppe**
**Studio Consulenza Brevettuale Via Rossini, 8**
**I-20122 Milan(IT)**

(54) Lyophilized amino acids for injectable use, in form of plastic bags.

㉗ Compositions comprising lyophilized amino acids for the parenteral alimentation, in form of bags of a suitable plastic material, are described.

EP 0 351 686 A2

## LYOPHILIZED AMINO ACIDS FOR INJECTABLE USE, IN FORM OF PLASTIC BAGS

The present invention relates to pharmaceutical compositions comprising lyophilized amino acids.

More particularly, the invention relates to bags made of suitable plastic material, containing sterile, apyrogenic lyophilized amino acids to be used for the parenteral alimentation either directly by infusion, after appropriate reconstitution with suitable solutions, or for the preparation of "tailored" solutions of various composition of nutritive substances (glycides, lipids, electrolytes, etc.).

Parenteral alimentation is a technique more and more used in hospital practice and it consists in administering through a peripheral or central vein nutritive substances which cannot be introduced by the natural digestive route.

Nutritive substances in order to be directly introduced into the blood stream, without affecting the organs involved in digestion, absorption and chemical modulation, have to fulfill restrictive requisites:
- they must be simple low molecular weight substances, such as amino acids, sugars, finely emulsified oils of particle size lower than 1 micron diameter;
- they must be vehicled in sterile, apyrogenic, low concentration aqueous solutions.

Parenteral alimentation is a therapy which depends on the requirements of each single patient: on the basis of the nutritional parameters, glycide and amino acid needs, water contribution, additions of electrolytes, vitamins, insulin, lipids and other specific drugs are calculated.

The "diet" composition is often formulated by means of suitably programmed computers.

There are no ready-to-use preparations of injectable grade, which contain all said nutritive substances admixed: the industrial production of said mixtures is made difficult by problems of chemico-physical incompatibility among the various components, which incompatibility is enhanced by the thermic sterilization processes.

On the contrary, the various ingredients are singly available in solutions of different concentrations, generally distributed in 500 ml glass bottles.

Parenteral alimentation must be carried out with great care, in order to secure the maximum effectiveness. Particularly, the infused liquid must contain all the dietary components and have a homogeneous composition.

The administration must be distributed uniformly during 24 hours at a constant rate.

In order to fulfill these requirements, the solutions containing the various ingredients were first administered by means of a multi-way defluxer (which, through a series of branches, allowed to draw the liquid at the same time from more bottles.

This techinque proved to be completely unfit, due to the difficulty in adjusting the flow from the various bottles and obtaining an homogeneous admixture.

From the technical point of view, the better results are represented by the so-called "administration bags", which are flexible transparent containers in plastic material, similar to the bags for human plasma, having a capacity of 1 to 5 liters. Said bags have openings closed by plugs or pierceable membranes and sometimes also have withdrawal and administration sets to allow the introduction and the aministration of liquids with no contacts with air, under aseptic conditions.

The various solutions constituting the daily diet of the patient are transferred from the various bottles into said bag, then admixed and administered during 24 hours, at a constant rate.

Even though this technique is optimal from the methodological point of view, it is very difficult to be carried out in practice since the operator must handle a great number of bottles.

Thus contamination risks increase and there is waste of product since incompletely exhausted bottles are not used for any subsequent preparation.

Due to the increasing needs for administration bags by the department of hospitals, it was necessary to simplify the work of the operator as much as possible, in view of safety reasons, greater productivities and a more rational use of the products.

Thus, bags of great capacity (5-10 liter volume) have been placed at the operators' disposal, which bags will be hereinafter called "mother bags", provided with suitable connections which allow to withdraw the liquid under aseptic conditions.

Said "mother bags" are filled with distilled water or concentrated sugar solutions (generally glucose) and thermically sterilized. By connecting said "mother bags" with the "administration bag", the exactly requested amounts of water and sugar are transferred into the diet formulation by means of a single operation.

This operation is further favoured by the availabilty of suited computerized devices provided with metering pumps which compute and automatically carry out the withdrawals from the various "mother bags".

However, electrolytes, lipids, amino acids presently are withdrawn from commercially available standard glass confections.

The present invention relates to a plastic bag containing mixtures of sterile apyrogenic amino acids, which are lyophilized, i.e. in form of an istantaneously soluble powder, instead of a solution.

The present invention, making easier the all procedures, favours and makes safer the operations for the preparation of bags for the parenteral alimentation.

In fact:

- in case of a confection containing large amounts of lyophilized product, a concentrated mother solution of amino acids is formed by means of just one procedure of dilution with water or glycide solutions, which mother solution will successively be distributed in the various administration bags;

- in case of bags containing single dosages, no more handlings are required as the bags themselves become the administration bags after addition of water and concentrated glycid solution which dissolve the lyophilized product with no changes in the water contribution.

Moreover, the use of sterile apyrogenic lyophilized products instead of solutions, allows to avoid the thermic sterilization of the product after the confection and therefore to use, as the container, plastic materials containing lower amounts of additives or even free from plasticizers which cannot support the high sterilization temperatures.

Therefore, the "administration bag" containing the lyophilized product, is suitable for receiving also lipid emulsions which usually cannot come into contact with materials containing plasticizers.

The confection according to the invention, being lighter and less bulky, is also more advantageous from the handling, transport and stocking point of views.

Finally, the use of a lyophilized product makes easier the problems connected with plastic release, allowing to achieve validity times for the confection higher than 3 years.

The amino acid mixtures according to the invention have compositions similar to the commercially available ones, which are used in parenteral alimentation in both not specific pathologies and in the special ones (nephropathies, septicaemias, hepatopathies, etc.) or in paediatrics.

The bags are of plastic material, according to law, completely free from plasticizers or containing a minimum amount thereof, and are fitted with the usual connections provided in the "administration bags" or in the "mother bags".

The bags containing the lyophilized product, with a further protection from moisture and light, can be manufactured in coupled sheets, of paper-alluminium-polythene or polythene-alluminium-polyester type.

The mixture of the lyophilized sterile apyrogenic amino acids is packed in sterile room in plastic material bags which have previously been sterilized by means of ethylene oxide gas or gamma rays.

The product is introduced through a suited feed opening, which is successively closed by a rubber plug and capsule, or through an opening made on a bag side and subsequently closed by heat seal. No thermal sterilizations are required after the packaging, as the product is a sterile lyophilized product packed under aseptic conditions.

The amino acid amount supplied in the bag depends on the capacity of the bag and on the intended use: in case it will be used for making a "mother bag", the amino acid content can also be 400 or 2.000 g; in case it will be used as an "administration bag", it will contain, for example, 50, 75, 100 or 120 g of the product, i.e. amounts which are usually employed for single administrations.

The following non limiting example further illustrates the present invention.

## EXAMPLE

Plastic bags of ethylene-vinyl acetate copolymer (EVA) dosed to 100 g of lyophilized amino acids.

| | |
|---|---|
| L-isoleucine | 6,2 g |
| L-leucine | 8,2 g |
| L-lysin (acetate) | 7,9 (11,15) g |
| L-methionine | 3,7 g |
| L-cysteine (HCl.H$_2$O) | 0,3 (0,435) g |
| L-phenylalanine | 4,9 g |
| L-tyrosine | 0,2 g |
| L-threonine | 5 g |
| L-tryptophan | 1,5 g |
| L-valine | 7,2 g |
| L-arginine | 10,5 g |
| L-histidine | 2,4 g |
| L-alanine | 12 g |
| L-aspartic acid | 2,7 g |
| L-glutamic acid | 4,8 g |
| glycine | 3,9 g |
| L-proline | 11,4 g |
| L-serine | 6,8 g |

## Claims

1. Bags of plastic material containing lyophilized sterile apyrogenic amino acids.

2. Bags as claimed in claim 1, containing 400 to 2.000 g of lyophilized amino acids.

3. Bags as claimed in claim 1, containing 50 to 120 g of lyophilized amino acids.

4. Bags as claimed in any one of the above claims, consisting of plastic material substantially free from plasticizers.